# FASCICULE DE BREVET EUROPEEN

(11) **EP 1 500 410 B1**
(45) Date de publication et mention de la délivrance du brevet: **09.01.2008**
(21) Numéro de dépôt: 04291862.3
(22) Date de dépôt: 22.07.2004
(51) Int. Cl.: A61N 1/32, A61N 1/40, A61N 2/02

(54) **Génération d'un champ électrique, magnétique, et/ou electromagnétique pulsé**
Vorrichtung zur Generierung von elektrischem, magnetischem und/oder pulsierendem elektromagnetischem Feld
Generation of an electric, magnetic and/or pulsed electromagnetic field

(30) Priorité: 22.07.2003 FR 0308945
(43) Date de publication de la demande: 26.01.2005
(73) Titulaire: Ecosystem Sarl, 75116 Paris (FR); 2EL spol. Sr. O., 504 01 Novy Bydzov (CZ)
(72) Inventeur: Lauterbach, Antonin, 50401 Novy Bydzov (CZ); Myslivec, Bohumil, 50006 Hradec Kralove (CZ); Pottecher, Frédéric, 28140 Terminiers (FR)
(74) Mandataire: Peaucelle, Chantal

(56) Documents cités:
- WO-A-91/04070
- GB-A- 1 479 734
- US-A- 4 977 895
- US-A1- 2002 055 762
- US-B1- 6 321 119

## Description

La présente invention concerne un procédé de génération d'un spectre de fréquence d'un champ électrique, magnétique et/ou électromagnétique pulsé et son application à la génération desdits champs dans des applications de traitement de certaines maladies.

Les cellules vivantes sont capables de réagir à des signaux électriques, magnétiques et/ou électromagnétiques. Ces signaux peuvent être utilisés d'un point de vue thérapeutique pour traiter des pathologies diverses, supprimer la douleur ou encore pour stimuler les muscles et de manière générale pour aider à retrouver une bonne condition physique.

Traditionnellement, on a recours à trois types de signaux : des signaux à fréquence fixe, c'est-à-dire dans lesquels les signaux sont émis à une fréquence qui ne varie pas durant toute la durée de la séance, des signaux qui balayent régulièrement un spectre entre une fréquence minimum et une fréquence maximum avec un incrément déterminé ou encore, en travaillant sur des fréquences fixes qui se répètent pendant une durée déterminée et qui changent aléatoirement de fréquence entre une fréquence minimum et une fréquence maximum tel que décrit dans US 2002/0055762 A1 par exemple.

Cependant, l'inconvénient de ces signaux utilisés traditionnellement est que les fréquences utilisées se répètent au fur et à mesure que la séance se prolonge. Cela a pour principal effet d'induire une accoutumance et les cellules deviennent de moins en moins réceptives aux signaux. Le traitement perd alors en efficacité et, comme ce sont toujours les mêmes fréquences qui sont utilisées, certaines cellules peuvent ne pas être réceptives, car on n'est pas sur la fréquence à laquelle elles répondent.

Le procédé objet de la présente invention permet de remédier à ces inconvénients et il s'applique aux trois types de signaux précédents.

A cette fin, le procédé de génération d'un spectre de fréquences d'un champ électrique, magnétique et/ou électromagnétique pulsé selon la présente invention se caractérise en ce que l'on effectue un tirage aléatoire des fréquences d'une série d'impulsions dans un intervalle de fréquences défini autour d'au moins une fréquence fixe selon une variation aléatoire déterminée.

Préférentiellement, ledit intervalle de fréquences est supérieur ou égal à 1 Hertz et inférieur ou égal à 2 Hertz.

Plus particulièrement, ledit intervalle de fréquences est centré sur ladite fréquence fixe.

Plus particulièrement encore, ladite fréquence fixe est inférieure à 100 Hertz.

Avantageusement, ladite variation aléatoire est d'une précision de l'ordre du milli Hertz.

Ainsi, avec la capacité de calcul des calculateurs actuels, une telle variation permet d'obtenir une couverture du champ proche de l'intégralité de l'intervalle de fréquences sur lequel il s'applique.

Particulièrement, la puissance desdites impulsions magnétiques est supérieure à 1 milli Tesla.

Plus avantageusement encore, on réitère au cours d'une même séance ledit tirage aléatoire en faisant varier ladite fréquence fixe selon un incrément déterminé.

Préférentiellement, ledit intervalle de fréquence correspond à au moins la valeur dudit incrément.

Plus préférentiellement, le procédé selon l'invention est mis en oeuvre pour plusieurs balayages successifs d'une gamme de fréquences déterminées inférieures à 100 Hertz.

Plus préférentiellement encore, lesdits balayages successifs sont mis en oeuvre pour une durée de l'ordre de 20 minutes.

Le procédé selon l'invention peut s'appliquer à la génération d'un champ magnétique pulsé.

De même, le procédé selon l'invention peut s'appliquer à la génération d'un champ électrique pulsé pour l'électrothérapie.

Enfin, le procédé selon l'invention peut s'appliquer à la génération d'un champ magnétique pulsé pour la magnétothérapie.

Dans le procédé objet de la présente invention, la fréquence des signaux générés est différente à chaque nouveau signal car on fait varier aléatoirement les fréquences des signaux dans la plage que l'on décide pour une fréquence fixe, par exemple plus ou moins 0.75 Hertz dans le cas d'une fréquence fixe et dans le cas des fréquences variables à balayage régulier ou aléatoire de fréquence en faisant varier la fréquence dans une plage comprise entre plus ou moins la moitié de la différence de fréquence entre deux signaux c'est-à-dire, si par exemple la fréquence varie de deux Hertz en deux Hertz, on fait varier aléatoirement la fréquence à l'intérieur d'un intervalle de plus ou moins 1 Hertz autour de la fréquence et ceci aléatoirement à chaque nouveau signal. En conséquence, balayage après balayage, on finit par recouvrir la totalité du spectre des fréquences comprises entre la fréquence minimum et la fréquence maximum, avec une précision qui ne dépend que de l'algorithme utilisé et de la puissance du calculateur. Ainsi, on est aujourd'hui en mesure d'obtenir des précisions de l'ordre d'un millième de Hertz.

Cette invention permet d'obtenir de meilleurs résultats thérapeutiques.
Le procédé selon l'invention permet également d'obtenir un résultat plus rapide et plus efficace. La mise en oeuvre du procédé selon l'invention nécessite donc moins de séances d'application ou alors des séances plus courtes.

Le procédé selon l'invention s'applique tout spécialement, et sans que cette liste ne soit exhaustive, dans les domaines de l'électrothérapie, dans lesquels les signaux généraux sont de type électrique, la magnétothérapie, dans lequel les signaux générés sont de type magnétique, et en particulier, à l'intérieur de cette thérapie dans le domaine des champs magnétiques pulsés. Dans ce domaine les meilleurs résultats sont obtenus avec des fréquences inférieures à cent Hertz et avec des puissances générées supérieures à 1 milli Tesla et des durées de séance inférieure à une heure. Des applications peuvent aussi être envisagées si les signaux sont de type lumineux comme les lasers.

L'invention sera mieux comprise à la lecture de la description détaillée ci après faite en référence aux dessins sur lesquels :
- la figure 1 représente l'enveloppe d'une impulsion, par exemple électromagnétique, généré à fréquence fixe,
- la figure 2 représente l'enveloppe d'une série d'impulsions générée en mettant en oeuvre le procédé selon l'invention autour de la même fréquence fixe,
- la figure 3 représente l'enveloppe d'une série d'impulsions, générée à différentes fréquences fixes,
- la figure 4 représente l'enveloppe obtenue pour la même série d'impulsions que pour la figure 3, mais en mettant oeuvre le procédé selon l'invention.

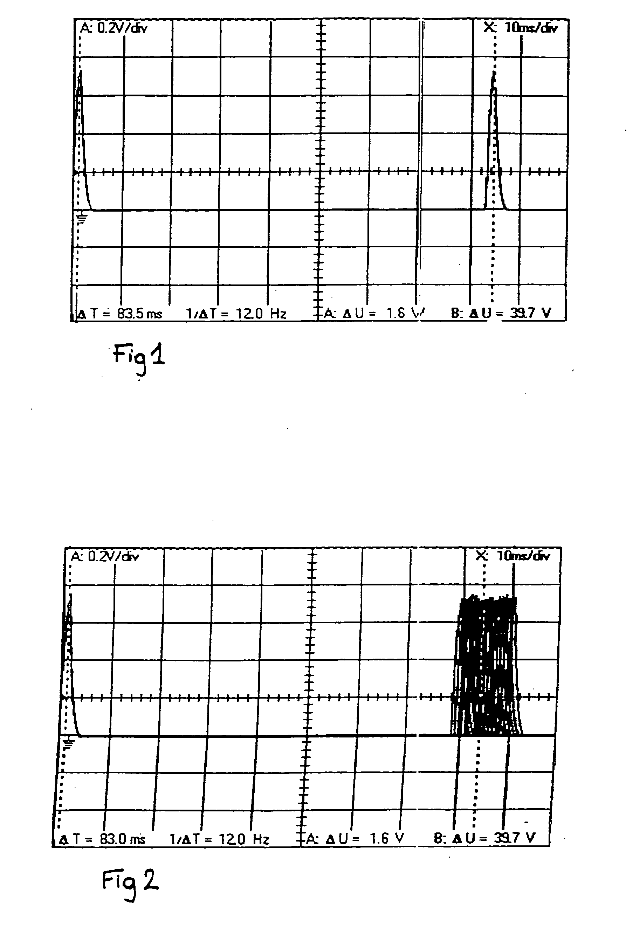

Sur la figure 1 est représentée une impulsion, par exemple magnétique, pour une fréquence fixe de 12 Hz d'une puissance de l'ordre de quelques milli Tesla.

Sur la figure 2, est représentée une série d'impulsion obtenue pour la même fréquence fixe en mettant en oeuvre le procédé selon l'invention.

On obtient ainsi un large spectre de fréquence généré par le procédé selon l'invention. Un tirage aléatoire des fréquences de la série d'impulsions est effectué dans un intervalle de fréquence défini d'une amplitude de 1,5 Hertz centré autour de la fréquence fixe de 12 Hertz et avec une variation aléatoire permettant une précision de l'ordre du milli Hertz.

On a ainsi couvert la quasi-totalité de la bande spectrale comprise entre 11,25 Hertz et 12,75 hertz.

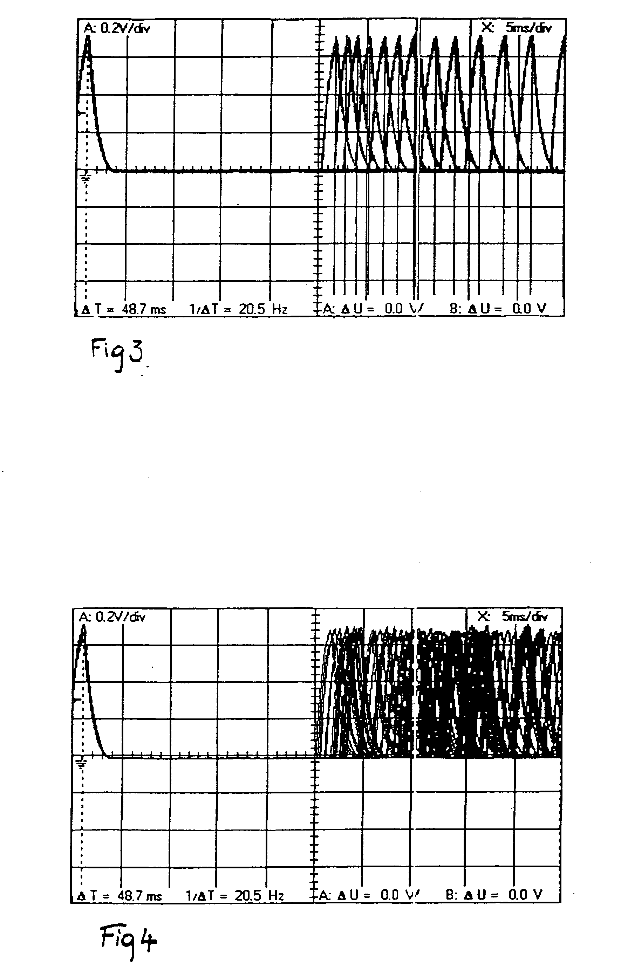

Sur la figure 3 est représentée une série d'impulsions obtenue pour un champ magnétique pulsé avec un incrément de fréquence de 1,5 Hertz. Les fréquences fixes générées sont ici comprises entre 40 Hertz et 22 Hertz.

Sur la figure 4 est représentée la même série d'impulsions obtenue en mettant en oeuvre le procédé selon l'invention autour de chaque fréquence fixe.

On obtient alors un balayage de fréquence quasi total pour des valeurs comprises entre 40,75 Hertz et 21,25 Hertz à la valeur près de la précision de la variation aléatoire qui est, aujourd'hui, de l'ordre du milli Hertz.

L'incrément reste ici de 1,5 Hertz et l'intervalle de fréquence sur lequel est mis en oeuvre le procédé est centré sur chacune des fréquences fixe des impulsions successives et a une amplitude de 1,5 Hertz équivalente à la valeur de l'incrément.

La mise en oeuvre de ce procédé sera particulièrement utile pour le traitement de nombreuses maladies pour lesquelles l'efficacité de l'application d'un champ électrique, magnétique et/ou électromagnétique pulsé a été mise en évidence et notamment dans les maladies de dégénérescence comme par exemple pour le traitement des rhumatismes, de la sclérose en plaque, de la maladie d'Alzheimer, de la maladie de Parkinson, de l'asthme ...

La mise en oeuvre de ce procédé sera également utile pour l'accélération des processus de réparation comme par exemple la réparation osseuse suite aux fractures...

## Revendications

1. Procédé de génération d'un spectre de fréquences d'un champ électrique, magnétique et/ou électromagnétique pulsé, **caractérisé en ce que** l'on effectue un tirage aléatoire des fréquences d'une série d'impulsions dans un intervalle de fréquences défini autour d'au moins une fréquence fixe selon une variation aléatoire déterminée.

2. Procédé selon la revendication 1, **caractérisé en ce que** ledit intervalle de fréquences est supérieur ou égal à 1 Hertz et inférieur ou égal à 2 Hertz.

3. Procédé selon l'une des revendications 1 ou 2, **caractérisé en ce que** ledit intervalle de fréquences est centré sur ladite fréquence fixe.

4. Procédé selon l'une quelconque des revendications 1 à 3, **caractérisé en ce que** ladite fréquence fixe est inférieure à 100 Hertz.

5. Procédé selon l'une quelconque des revendications 1 à 4, **caractérisé en ce que** ladite variation aléatoire est d'une précision de l'ordre du milli Hertz.

6. Procédé selon l'une quelconque des revendications 1 à 5, **caractérisé en ce que** la puissance desdites impulsions magnétiques est supérieure à 1 milli Tesla.

7. Procédé selon l'une quelconque des revendications 1 à 6, **caractérisé en ce que** l'on réitère au cours d'une même séance ledit tirage aléatoire en faisant varier ladite fréquence fixe selon un incrément déterminé.

8. Procédé selon la revendication 7, **caractérisé en ce que** ledit intervalle de fréquence correspond à au moins la valeur dudit incrément.

9. Procédé selon l'une des revendications 7 ou 8, **caractérisé en ce qu**'il est mis en oeuvre pour plusieurs balayages successifs d'une gamme de fréquences déterminées inférieures à 100 Hertz.

10. Procédé selon la revendication 9, **caractérisé en ce que** lesdits balayages successifs sont mis en oeuvre pour une durée de l'ordre de 20 minutes.

11. Application du procédé selon l'une quelconque des revendications 1 à 10 à la génération d'un champ magnétique pulsé, d'un champ électrique pulsé ou d'un champ électromagnétique pulsé.

## Claims

1. Method for the generation of a frequency spectrum of a pulsed electric, magnetic and/or electromagnetic field, **characterized in that** a random selection of frequencies is made from a series of pulses in a frequency interval defined around a fixed frequency according to a given random variation.

2. Method according to claim 1, **characterized in that** said frequency interval is greater than or equal to 1 Hertz and is less than or equal to 2 Hertz.

3. Method according to one of claims 1 or 2, **characterized in that** said frequency interval is centred on said fixed frequency.

4. Method according to any one of claims 1 to 3, **characterized in that** said fixed frequency is less than 100 Hertz.

5. Method according to any one of claims 1 to 4, **characterized in that** said random variation has a precision of the order of a milli Hertz.

6. Method according to any one of claims 1 to 5, **characterized in that** the power of said magnetic pulses is greater than 1 milli Tesla.

7. Method according to any one of claims 1 to 6, **characterized in that** during the same session, said random selection is repeated, varying said fixed frequency according to a given increment.

8. Method according to claim 7, **characterized in that** said frequency interval corresponds to at least the value of said increment.

9. Method according to one of claims 7 or 8, **characterized in that** it is implemented for several successive scans of a range of given frequencies of less than 100 Hertz.

10. Method according to claim 9, **characterized in that** said successive scans are implemented for a duration of the order of 20 minutes.

11. Application of the method according to any one of claims 1 to 10 to the generation of a pulsed magnetic field, a pulsed electric field or a pulsed electromagnetic field.

## Patentansprüche

1. Verfahren zur Erzeugung eines Frequenzspektrums eines pulsierenden elektrischen, magnetischen und/oder elektromagnetischen Feldes, **dadurch gekennzeichnet, dass** eine zufällige Anregung von Frequenzen einer Reihe von Impulsen in einem Frequenzintervall, das um mindestens eine feste Frequenz herum definiert ist, nach einer bestimmten zufälligen Variation bewirkt wird.

2. Verfahren nach Anspruch 1, **dadurch gekennzeichnet, dass** das Frequenzintervall größer oder gleich 1 Hertz und kleiner oder gleich 2 Hertz ist.

3. Verfahren nach einem der Ansprüche 1 oder 2, **dadurch gekennzeichnet, dass** das Frequenzintervall auf die feste Frequenz zentriert ist.

4. Verfahren nach einem der Ansprüche 1 bis 3, **dadurch gekennzeichnet, dass** die feste Frequenz kleiner als 100 Hertz ist.

5. Verfahren nach einem der Ansprüche 1 bis 4, **dadurch gekennzeichnet, dass** die zufällige Variation eine Genauigkeit in der Größenordnung von Millihertz aufweist.

6. Verfahren nach einem der Ansprüche 1 bis 5, **dadurch gekennzeichnet, dass** die Stärke der magnetischen Impulse größer als 1 Millitesla ist.

7. Verfahren nach einem der Ansprüche 1 bis 6, **dadurch gekennzeichnet, dass** im Verlauf der gleichen Sitzung die zufällige Anregung wiederholt wird, wobei die feste Frequenz um ein bestimmtes Inkrement verändert wird.

8. Verfahren nach Anspruch 7, **dadurch gekennzeichnet, dass** das Frequenzintervall mindestens dem Betrag des Inkrements entspricht.

9. Verfahren nach einem der Ansprüche 7 oder 8, **dadurch gekennzeichnet, dass** es über mehrere aufeinanderfolgende Abtastungen eines Bereichs bestimmter Frequenzen durchgeführt wird, die unter 100 Hertz liegen.

10. Verfahren nach Anspruch 9, **dadurch gekennzeichnet, dass** die aufeinanderfolgenden Abtastungen über einen Zeitraum in der Größenordnung von 20 Minuten durchgeführt werden.

11. Verwendung des Verfahrens nach einem der Ansprüche 1 bis 10 zur Erzeugung eines pulsierenden Magnetfeldes, eines pulsierenden elektrischen Feldes oder eines pulsierenden elektromagnetischen Feldes.
